# EUROPEAN PATENT APPLICATION

(11) **EP 3 711 558 A1**
(43) Date of publication of application: **23.09.2020**
(21) Application number: 19020190.5
(22) Date of filing: 22.03.2019
(51) Int. Cl.: A24F 47/00

(54) **SMOKING SUBSTITUTE SYSTEM**

(71) Applicant: NERUDIA LIMITED, Liverpool Merseyside L24 9HP (GB)
(72) Inventor: The designation of the inventor has not yet been filed
(74) Representative: Mewburn Ellis LLP

(57) **Abstract**

A heat-not-burn device comprising: a heater configured to operate at a first temperature in a first heating mode and a second temperature in a second heating mode, the second temperature is higher than the first temperature; and a sensor configured to measure an ambient temperature; wherein the heat-not-burn device is configured to disable the second heating mode when the measured ambient temperature exceeds a predetermined threshold.

## Description

### TECHNICAL FIELD

The present invention relates to a smoking substitute system and particularly, although not exclusively, to a smoking substitute system comprising a heat-not-burn device and method for controlling operation of the heat-not-burn device based on a measured ambient temperature.

### BACKGROUND

The smoking of tobacco is generally considered to expose a smoker to potentially harmful substances. It is generally thought that a significant amount of the potentially harmful substances are generated through the heat caused by the burning and/or combustion of the tobacco and the constituents of the burnt tobacco in the tobacco smoke itself.

Conventional combustible smoking articles, such as cigarettes, typically comprise a cylindrical rod of tobacco comprising shreds of tobacco which is surrounded by a wrapper, and usually also a cylindrical filter axially aligned in an abutting relationship with the wrapped tobacco rod. The filter typically comprises a filtration material which is circumscribed by a plug wrap. The wrapped tobacco rod and the filter are joined together by a wrapped band of tipping paper that circumscribes the entire length of the filter and an adjacent portion of the wrapped tobacco rod. A conventional cigarette of this type is used by lighting the end opposite to the filter, and burning the tobacco rod. The smoker receives mainstream smoke into their mouth by drawing on the mouth end or filter end of the cigarette.

Combustion of organic material such as tobacco is known to produce tar and other potentially harmful by-products. There have been proposed various smoking substitute systems (or "substitute smoking systems") in order to avoid the smoking of tobacco.

Such smoking substitute systems can form part of nicotine replacement therapies aimed at people who wish to stop smoking and overcome a dependence on nicotine.

Smoking substitute systems include electronic systems that permit a user to simulate the act of smoking by producing an aerosol (also referred to as a "vapour") that is drawn into the lungs through the mouth (inhaled) and then exhaled. The inhaled aerosol typically bears nicotine and/or flavourings without, or with fewer of, the odour and health risks associated with traditional smoking.

In general, smoking substitute systems are intended to provide a substitute for the rituals of smoking, whilst providing the user with a similar experience and satisfaction to those experienced with traditional smoking and with combustible tobacco products. Some smoking substitute systems use smoking substitute articles (also referred to as a "consumables") that are designed to resemble a traditional cigarette and are cylindrical in form with a mouthpiece at one end.

The popularity and use of smoking substitute systems has grown rapidly in the past few years. Although originally marketed as an aid to assist habitual smokers wishing to quit tobacco smoking, consumers are increasingly viewing smoking substitute systems as desirable lifestyle accessories.

There are a number of different categories of smoking substitute systems, each utilising a different smoking substitute approach.

One approach for a smoking substitute system is the so-called Heated Tobacco ("HT") approach in which tobacco (rather than an "e-liquid") is heated or warmed to release vapour. HT is also known as "heat not burn" ("HNB"). The tobacco may be leaf tobacco or reconstituted tobacco. The vapour may contain nicotine and/or flavourings. In the HT approach the intention is that the tobacco is heated but not burned, i.e. the tobacco does not undergo combustion.

A typical HT smoking substitute system may include a device and a consumable. The consumable may include the tobacco material. The device and consumable may be configured to be physically coupled together. In use, heat may be imparted to the tobacco material by a heating element of the device, wherein airflow through the tobacco material causes components in the tobacco material to be released as vapour. A vapour may also be formed from a carrier in the tobacco material (this carrier may for example include propylene glycol and/or vegetable glycerine) and additionally volatile compounds released from the tobacco. The released vapour may be entrained in the airflow drawn through the tobacco.

As the vapour passes through the consumable (entrained in the airflow) from the location of vaporisation to an outlet of the consumable (e.g. a mouthpiece), the vapour cools and condenses to form an aerosol for inhalation by the user. The aerosol will normally contain the volatile compounds.

In HT smoking substitute systems, heating as opposed to burning the tobacco material is believed to cause fewer, or smaller quantities, of the more harmful compounds ordinarily produced during smoking. Consequently, the HT approach may reduce the odour and/or health risks that can arise through the burning, combustion and pyrolytic degradation of tobacco.

There may be a need for improved design of smoking substitute systems, in particular HT smoking substitute systems, to enhance the user experience and improve the function of the HT smoking substitute system.

The present disclosure has been devised in the light of the above considerations.

### SUMMARY OF THE INVENTION

Aerosol generation in heat-not-burn (HNB) may be closely relate to the operating temperature of the heater, e.g. a temperature at the surface of the heating element during its operation. A higher operating temperature may allow more volatile to be vaporised and thereby increases the amount of aerosol to be formed with each puff. Furthermore, a higher operating temperature may result in a more intense flavour in the aerosol. A HNB device may be provided that allows a user to switch between different operating temperatures according to one's needs. However, operating at a higher temperature may be detrimental in some situations, e.g. at warmer climates. For example, there may be too much aerosol being generated in a puff, or with an overly intense flavour.

At its most general, the present invention relates to a heat-not-burn device configured to operate at two different heating modes, wherein the device disable one of the two heating modes based on detected ambient temperature.

According to a first aspect of the present invention, there is provided a heat-not-burn (HNB) device comprising:
a heater configured to operate at a first temperature in a first heating mode and a second temperature in a second heating mode, the second temperature is higher than the first temperature; and
a sensor configured to measure an ambient temperature;
wherein the heat-not-burn device is configured to disable the second heating mode when the measured ambient temperature exceeds a predetermined threshold.

The first heating mode may be defined as a normal operating mode, e.g. the heater may operate at the first temperature which is a typical operating temperature. The first heating mode may be an operating mode where the consumable is heated at a standard operating temperature. The second heating mode may be defined as a boost operating mode, e.g. the heater may operate at the second temperature which is higher than the first temperature. When operating in the second heating mode, the consumable may be able to reach the desired temperature quickly and/or being heated at a higher temperature and thereby increases the rate of aerosol generation.

The first temperature may range from 300°C to 330°C, preferably the first temperature may range from 305°C to 325°C, and may be 315°C. The second temperature may range from 330°C to 360°C, preferably the second temperature may range from 335°C to 355°C, and may be 345°C.

The predetermined threshold may be at least 40°C, e.g. when a monoacetate filter is used in the aerosol-forming article. Alternatively, the predetermined threshold may range from 35°C-40°C, e.g. when a hollow bore is used instead of the monoacetate filter in the aerosol-forming article.

However, during warmer seasons or at locations with tropical climate, the ambient temperature may be so high that when the heater operates at the second temperature it may overheat the consumable, e.g. it may unfavourably causes the consumable to generate too much aerosol or to produce undesired volatiles. Therefore advantageously, by disabling the second operating mode once the ambient temperature exceeds a predetermined threshold or predetermined threshold temperature, the device may be prevented from generating undesired volatiles. Additionally, the risk of overheating at the heater may be greatly reduced.

Optional features will now be set out. These are applicable singly or in any combination with any aspect.

Optionally, the device may comprise a controller electrically connected with the heater and the sensor, the controller is configured to receive the measured ambient temperature from the sensor and to disable the second heating mode upon determining that said measured ambient temperature exceeds the pre-determined threshold. The sensor may be configured to continuously measure the ambient temperature and therefrom feeds the measured ambient temperature to the controller. Therefrom the controller may be configured to compare said measured ambient temperature with a predetermined threshold temperature in a device storage. If the measured ambient temperature is determined to have exceeded the pre-determined threshold the controller may disable the second heating mode. Thus, the controller may be configured to intelligentially control the heating of the consumable based on ambient temperature.

Optionally, the controller may be configured to disable the second heating mode by switching the operation of the heater from the second heating mode to the first heating mode. That is, the heater is not switched off but instead it operates at a lowered temperature. This may ensure the device continuously to generate aerosol.

Alternatively, the controller may be configured to disable the second heating mode by disabling the heater altogether. That is, the controller may cease energising the heater so as to allow the heater and/or consumable to cool down. The heater may resume operating in a first operating mode once the device receives a user input, e.g. to switch on the device, or after a predetermined period of time, e.g. 30 seconds.

Optionally, the device further comprises an output means configured to output one or more of a haptic feedback, an audio feedback and a visual feedback upon disabling of the second heating mode. For example, the output means may be a haptic device for providing said haptic feedback. The output means may be a buzzer or a speaker for providing audio feedback. The output means may be an LED, an array of LED, or any other visual indicators known to the person skilled in the art. Upon disabling the second heating mode, the controller may activate the output means for outputting one or more of the haptic feedback, the audio feedback and the visual feedback. In some embodiment, the output means may be used to indicate the user that the ambient temperature of the device is too high and thus second heating mode has been disabled. In this way, a versatile device is provided which is able to inform the user of second heating mode disabled.

Optionally, further comprises a user interface, wherein upon receiving a user input at the user interface the heater is configured to switch between operating in the second heating mode and the first heating mode. This may advantageously allow the user to manually specify the desired operating temperature. For example, the controller may be configured to receive the user input, via the user interface, to switch to the second heating mode from the first heating mode. The controller may further generate a feedback, via the output means, indicating the user that the second heating mode is not enabled if the controller detected that the ambient temperature exceeds the predetermined threshold.

Optionally, the heater is prevented from switching from operating in the first operating mode to the second operating mode if the measured ambient temperature exceeds the predetermined threshold. Optionally, the device is configured to, via an output means, output one or more of a haptic feedback, an audio feedback and a visual feedback when the heater is prevented from switching from operating in the first operating mode to the second operating mode. That is when the measured ambient temperature exceeds the predetermined threshold, the controller prevents the user from manually switching to second operating mode, and thereby informs the user.

The device may comprise an elongate body. An end of the elongate body may be configured for engagement with an aerosol-forming article. For example, the body may be configured for engagement with a heated tobacco (HT) consumable (or heat-not-burn (HNB) consumable). The terms "heated tobacco" and "heat-not-burn" are used interchangeably herein to describe a consumable that is of the type that is heated rather than combusted (or are used interchangeably to describe a device for use with such a consumable). The device may comprise a cavity that is configured for receipt of at least a portion of the consumable (i.e. for engagement with the consumable). The aerosol-forming article may be of the type that comprises an aerosol former (e.g. carried by an aerosol-forming substrate).

The device may comprise a heater for heating the aerosol-forming article. The heater may comprise a heating element, which may be in the form of a rod that extends from the body of the device. The heating element may extend from the end of the body that is configured for engagement with the aerosol-forming article. In an embodiment, the heater is configured to operate in dual heating mode. Precisely, the heater is configured to operate at a first temperature in a first heating mode and at a second temperature in the second heating mode, wherein the second temperature being higher than the first temperature.

The heater (and thus the heating element) may be rigidly mounted to the body. The heating element may be elongate so as to define a longitudinal axis and may, for example, have a transverse profile (i.e. transverse to a longitudinal axis of the heating element) that is substantially circular (i.e. the heating element may be generally cylindrical). Alternatively, the heating element may have a transverse profile that is rectangular (i.e. the heater may be a "blade heater"). The heating element may alternatively be in the shape of a tube (i.e. the heater may be a "tube heater"). The heating element may take other forms (e.g. the heating element may have an elliptical transverse profile). The shape and/or size (e.g. diameter) of the transverse profile of the heating element may be generally consistent for the entire length (or substantially the entire length) of the heating element.

The heating element may be between 15 mm and 25 mm long, e.g. between 18 mm and 20 mm long, e.g. around 19 mm long. The heating element may have a diameter of between 1.5 mm and 2.5 mm, e.g. a diameter between 2 mm and 2.3 mm, e.g. a diameter of around 2.15 mm.

The heating element may be formed of ceramic. The heating element may comprise a core (e.g. a ceramic core) comprising Al2O3. The core of the heating element may have a diameter of 1.8 mm to 2.1 mm, e.g. between 1.9 mm and 2 mm. The heating element may comprise an outer layer (e.g. an outer ceramic layer) comprising Al2O3. The thickness of the outer layer may be between 160 µm and 220 µm, e.g. between 170 µm and 190 µm, e.g. around 180 µm. The heating element may comprise a heating track, which may extend longitudinally along the heating element. The heating track may be sandwiched between the outer layer and the core of the heating element. The heating track may comprise tungsten and/or rhenium. The heating track may have a thickness of around 20 µm.

The heating element may be located in the cavity (of the device), and may extend (e.g. along a longitudinal axis) from an internal base of the cavity towards an opening of the cavity. The length of the heating element (i.e. along the longitudinal axis of the heater) may be less than the depth of the cavity. Hence, the heating element may extend for only a portion of the length of the cavity. That is, the heating element may not extend through (or beyond) the opening of the cavity.

The heating element may be configured for insertion into an aerosol-forming article (e.g. a HT consumable) when an aerosol-forming article is received in the cavity. In that respect, a distal end (i.e. distal from a base of the heating element where it is mounted to the device) of the heating element may comprise a tapered portion, which may facilitate insertion of the heating element into the aerosol-forming article. The heating element may fully penetrate an aerosol-forming article when the aerosol-forming article is received in the cavity. That is, the entire length, or substantially the entire length, of the heating element may be received in the aerosol-forming article.

The heating element may have a length that is less than, or substantially the same as, an axial length of an aerosol-forming substrate forming part of an aerosol-forming article (e.g. a HT consumable). Thus, when such an aerosol-forming article is engaged with the device, the heating element may only penetrate the aerosol-forming substrate, rather than other components of the aerosol-forming article. The heating element may penetrate the aerosol-forming substrate for substantially the entire axial length of the aerosol forming-substrate of the aerosol-forming article. Thus, heat may be transferred from (e.g. an outer circumferential surface of) the heating element to the surrounding aerosol-forming substrate, when penetrated by the heating element. That is, heat may be transferred radially outwardly (in the case of a cylindrical heating element) or e.g. radially inwardly (in the case of a tube heater).

Where the heater is a tube heater, the heating element of the tube heater may surround at least a portion of the cavity. When the portion of the aerosol-forming article is received in the cavity, the heating element may surround a portion of the aerosol-forming article (i.e. so as to heat that portion of the aerosol-forming article). In particular, the heating element may surround an aerosol forming substrate of the aerosol-forming article. That is, when an aerosol-forming article is engaged with the device, the aerosol forming substrate of the aerosol-forming article may be located adjacent an inner surface of the (tubular) heating element. When the heating element is activated, heat may be transferred radially inwardly from the inner surface of the heating element to heat the aerosol forming substrate.

The cavity may comprise a (e.g. circumferential) wall (or walls) and the (tubular) heating element may extend around at least a portion of the wall(s). In this way, the wall may be located between the inner surface of the heating element and an outer surface of the aerosol-forming article. The wall (or walls) of the cavity may be formed from a thermally conductive material (e.g. a metal) to allow heat conduction from the heating element to the aerosol-forming article. Thus, heat may be conducted from the heating element, through the cavity wall (or walls), to the aerosol-forming substrate of an aerosol-forming article received in the cavity.

In some embodiments the device may comprise a cap disposed at the end of the body that is configured for engagement with an aerosol-forming article. Where the device comprises a heater having a heating element, the cap may at least partially enclose the heating element. The cap may be moveable between an open position in which access is provided to the heating element, and a closed position in which the cap at least partially encloses the heating element. The cap may be slideably engaged with the body of the device, and may be slideable between the open and closed positions.

The cap may define at least a portion of the cavity of the device. That is, the cavity may be fully defined by the cap, or each of the cap and body may define a portion of the cavity. Where the cap fully defines the cavity, the cap may comprise an aperture for receipt of the heating element into the cavity (when the cap is in the closed position). The cap may comprise an opening to the cavity. The opening may be configured for receipt of at least a portion of an aerosol-forming article. That is, an aerosol-forming article may be inserted through the opening and into the cavity (so as to be engaged with the device).

The cap may be configured such that when an aerosol-forming article is engaged with the device (e.g. received in the cavity), only a portion of the aerosol-forming article is received in the cavity. That is, a portion of the aerosol-forming article (not received in the cavity) may protrude from (i.e. extend beyond) the opening. This (protruding) portion of the aerosol-forming article may be a terminal (e.g. mouth) end of the aerosol-forming article, which may be received in a user's mouth for the purpose of inhaling aerosol formed by the device.

The device may comprise a power source or may be connectable to a power source (e.g. a power source separate to the device). The power source may be electrically connectable to the heater. In that respect, altering (e.g. toggling) the electrical connection of the power source to the heater may affect a state of the heater. For example, toggling the electrical connection of the power source to the heater may toggle the heater between an on state and an off state. The power source may be a power store. For example, the power source may be a battery or rechargeable battery (e.g. a lithium ion battery).

The device may comprise an input connection (e.g. a USB port, Micro USB port, USB-C port, etc.). The input connection may be configured for connection to an external source of electrical power, such as a mains electrical supply outlet. The input connection may, in some cases, be used as a substitute for an internal power source (e.g. battery or rechargeable battery). That is, the input connection may be electrically connectable to the heater (for providing power to the heater). Hence, in some forms, the input connection may form at least part of the power source of the device.

Where the power source comprises a rechargeable power source (such as a rechargeable battery), the input connection may be used to charge and recharge the power source.

The device may comprise a user interface (UI). In some embodiments the UI may include input means to receive operative commands from the user. The input means of the UI may allow the user to control at least one aspect of the operation of the device. In some embodiments the input means may comprise a power button to switch the device between an on state and an off state. In some embodiment, the input means of the UI may allow a user to switch the device between the first heating mode and the second heating mode.

In some embodiments the UI may additionally or alternatively comprise output means to convey information to the user. In some embodiments, the output means may be configured to indicate the user that the second heating mode is disabled if the ambient temperature is determined to have exceeded a predetermined threshold temperature. In some embodiments the output means may comprise a light to indicate a condition of the device (and/or the aerosol-forming article) to the user. The condition of the device (and/or aerosol-forming article) indicated to the user may comprise a condition indicative of the operation of the heater. For example, the condition may comprise whether the heater is in an off state or an on state. In some embodiments, the UI unit may comprise at least one of a button, a display, a touchscreen, a switch, a light, and the like. For example, the output means may comprise one or more (e.g. two, three, four, etc.) light-emitting diodes ("LEDs") that may be located on the body of the device.

The device may further comprise a puff sensor (e.g. airflow sensor), which form part of the input means of the Ul. The puff sensor may be configured to detect a user drawing on an end (i.e. a terminal (mouth) end) of the aerosol-forming article. The puff sensor may, for example, be a pressure sensor or a microphone. The puff sensor may be configured to produce a signal indicative of a puff state. The signal may be indicative of the user drawing (an aerosol from the aerosol-forming article) such that it is e.g. in the form of a binary signal. Alternatively or additionally, the signal may be indicative of a characteristic of the draw (e.g. a flow rate of the draw, length of time of the draw, etc.).

The device may comprise a controller or may be connectable to a controller that may be configured to control at least one function of the device. The controller may comprise a microcontroller that may e.g. be mounted on a printed circuit board (PCB). The controller may also comprise a memory, e.g. non-volatile memory. The memory may include instructions, which, when implemented, may cause the controller to perform certain tasks or steps of a method. Where the device comprises an input connection, the controller may be connected to the input connection.

The controller may be electrically connected to the heater and a sensor, and may be configured to control the operation of the heater (and e.g. the heating element) based on measurement of the sensor. In one example, the sensor is a temperature sensor to detect the ambient temperature of the device. In one aspect, the controller may be configured to receive the measured ambient temperature from the sensor and disable the second heating mode upon determining that the ambient temperature exceeds the predetermined threshold temperature. Thus, the controller may be configured to control vaporisation of an aerosol forming part of an aerosol-forming article engaged with the device. The controller may be configured to control the voltage applied by power source to the heater. For example, the controller may be configured to toggle between applying a full output voltage (of the power source) to the heater and applying no voltage to the heater. Alternatively or additionally, the control unit may implement a more complex heater control protocol.

The device may further comprise a voltage regulator to regulate the output voltage supplied by the power source to form a regulated voltage. The regulated voltage may subsequently be applied to the heater. In some embodiment, the voltage regulator may be used to control the output voltage supplied to the heater to operate in one of the first heating mode and the second heating mode based on ambient temperature measurement.

In some embodiments, where the device comprises a UI, the controller may be operatively connected to one or more components of the UI. The controller may be configured to receive command signals from an input means of the UI. The controller may be configured to control the heater in response to the command signals. For example, the controller may be configured to receive "on" and "off" command signals from the UI and, in response, may control the heater so as to be in a corresponding on or off state. Further, the controller may be configured to receive a command for controlling the heater or the device to switch from the first heating mode to the second heating mode via user input means. In response, the controller may be configured to indicate, through the output means, that the second heating mode is disabled if the controller detects that the ambient temperature exceeds the predetermined threshold temperature.

The controller may be configured to send output signals to a component of the UI. The UI may be configured to convey information to a user, via an output means, in response to such output signals (received from the controller). For example, where the device comprises one or more LEDs, the LEDs may be operatively connected to the controller. Hence, the controller may configured to control the illumination of the LEDs (e.g. in response to an output signal). For example, the controller may be configured to control the illumination of the LEDs according to (e.g. an on or off) state of the heater. In another example, the controller may be configured to control the illumination of the LEDs indicating that the second heating mode is disabled. Further, the controller may be configured to indicate the user that the second heating mode disabled through other output means such as haptic sensor and audio sensor etc.

Where the device comprises a sensor (e.g. a puff/airflow sensor), the controller may be operatively connected to the sensor. The controller may be configured to receive a signal from the sensor (e.g. indicative of a condition of the device and/or engaged aerosol-forming article). The controller may be configured to control the heater, or an aspect of the output means, based on the signal from the sensor.

The device may comprise a wireless interface configured to communicate wirelessly (e.g. via Bluetooth (e.g. a Bluetooth low-energy connection) or WiFi) with an external device. Similarly, the input connection may be configured for wired connection to an external device so as to provide communication between the device and the external device.

The external device may be a mobile device. For example, the external device may be a smart phone, tablet, smart watch, or smart car. An application (e.g. app) may be installed on the external device (e.g. mobile device). The application may facilitate communication between the device and the external device via the wired or wireless connection.

The wireless or wired interface may be configured to transfer signals between the external device and the controller of the device. In this respect, the controller may control an aspect of the device in response to a signal received from an external device. Alternatively or additionally, an external device may respond to a signal received from the device (e.g. from the controller of the device).

In a second aspect, there is provided a system (e.g. a smoking substitute system) comprising a device according to the first aspect and an aerosol-forming article. The aerosol-forming article may comprise an aerosol-forming substrate at an upstream end of the aerosol-forming article. The article may be in the form of a smoking substitute article, e.g. heated tobacco (HT) consumable (also known as a heat-not-burn (HNB) consumable).

As used herein, the terms "upstream" and "downstream" are intended to refer to the flow direction of the vapour/aerosol i.e. with the downstream end of the article/consumable being the mouth end or outlet where the aerosol exits the consumable for inhalation by the user. The upstream end of the article/consumable is the opposing end to the downstream end.

The aerosol-forming substrate is capable of being heated to release at least one volatile compound that can form an aerosol. The aerosol-forming substrate may be located at the upstream end of the article/consumable.

In order to generate an aerosol, the aerosol-forming substrate comprises at least one volatile compound that is intended to be vaporised/aerosolised and that may provide the user with a recreational and/or medicinal effect when inhaled. Suitable chemical and/or physiologically active volatile compounds include the group consisting of: nicotine, cocaine, caffeine, opiates and opoids, cathine and cathinone, kavalactones, mysticin, beta-carboline alkaloids, salvinorin A together with any combinations, functional equivalents to, and/or synthetic alternatives of the foregoing.

The aerosol-forming substrate may comprise plant material. The plant material may comprise least one plant material selected from the list including *Amaranthus dubius, Arctostaphylos uva-ursi* (Bearberry), *Argemone mexicana, Amica, Artemisia vulgaris,* Yellow Tees, *Galea zacatechichi, Canavalia maritima* (Baybean), *Cecropia mexicana* (Guamura), *Cestrum noctumum, Cynoglossum virginianum* (wild comfrey), *Cytisus scoparius, Damiana, Entada rheedii, Eschscholzia califomica* (California Poppy), *Fittonia albivenis, Hippobroma longiflora, Humulus japonica* (Japanese Hops), *Humulus lupulus* (Hops), *Lactuca virosa* (Lettuce Opium), *Laggera alata, Leonotis leonurus, Leonurus cardiaca* (Motherwort), *Leonurus sibiricus* (Honeyweed), *Lobelia cardinalis, Lobelia inflata* (Indian-tobacco), *Lobelia siphilitica, Nepeta cataria* (Catnip), *Nicotiana species* (Tobacco), *Nymphaea alba* (White Lily), *Nymphaea caerulea* (Blue Lily), Opium poppy, *Passiflora incamata* (Passionflower), *Pedicularis densiflora* (Indian Warrior), *Pedicularis groenlandica* (Elephant's Head), *Salvia divinorum, Salvia dorrii* (Tobacco Sage), Salvia species (Sage), *Scutellaria galericulata, Scutellaria lateriflora, Scutellaria nana, Scutellaria* species (Skullcap), *Sida acuta* (Wireweed), *Sida rhombifolia, Silene capensis, Syzygium aromaticum* (Clove), *Tagetes lucida* (Mexican Tarragon), *Tarchonanthus camphoratus, Tumera diffusa* (Damiana), *Verbascum* (Mullein), *Zamia latifolia* (Maconha Brava) together with any combinations, functional equivalents to, and/or synthetic alternatives of the foregoing.

The plant material may be tobacco. Any type of tobacco may be used. This includes, but is not limited to, flue-cured tobacco, burley tobacco, Maryland Tobacco, dark-air cured tobacco, oriental tobacco, dark-fired tobacco, perique tobacco and rustica tobacco. This also includes blends of the above mentioned tobaccos.

The tobacco may comprise one or more of leaf tobacco, stem tobacco, tobacco powder, tobacco dust, tobacco derivatives, expanded tobacco, homogenised tobacco, shredded tobacco, extruded tobacco, cut rag tobacco and/or reconstituted tobacco (e.g. slurry recon or paper recon).

The aerosol-forming substrate may comprise a gathered sheet of homogenised (e.g. paper/slurry recon) tobacco or gathered shreds/strips formed from such a sheet.

The aerosol-forming substrate may comprise one or more additives selected from humectants, flavourants, fillers, aqueous/non-aqueous solvents and binders.

The flavourant may be provided in solid or liquid form. It may include menthol, liquorice, chocolate, fruit flavour (including e.g. citrus, cherry etc.), vanilla, spice (e.g. ginger, cinnamon) and tobacco flavour. The flavourant may be evenly dispersed throughout the aerosol-forming substrate or may be provided in isolated locations and/or varying concentrations throughout the aerosol-forming substrate.

The aerosol-forming substrate may be formed in a substantially cylindrical shape such that the article/consumable resembles a conventional cigarette. It may have a diameter of between 5 and 10mm e.g. between 6 and 9mm or 6 and 8mm e.g. around 7 mm. It may have an axial length of between 10 and 15mm e.g. between 11 and 14mm such as around 12 or 13mm.

The article/consumable may comprise at least one filter element. There may be a terminal filter element at the downstream/mouth end of the article/consumable.

The or at least one of the filter element(s) (e.g. the terminal filter element) may be comprised of cellulose acetate or polypropylene tow. The at least one filter element (e.g. the terminal filter element) may be comprised of activated charcoal. The at least one filter element (e.g. the terminal element) may be comprised of paper. The or each filter element may be at least partly (e.g. entirely) circumscribed with a plug wrap e.g. a paper plug wrap.

The terminal filter element (at the downstream end of the article/consumable) may be joined to the upstream elements forming the article/consumable by a circumscribing tipping layer e.g. a tipping paper layer. The tipping paper may have an axial length longer than the axial length of the terminal filter element such that the tipping paper completely circumscribes the terminal filter element plus the wrapping layer surrounding any adjacent upstream element.

In some embodiments, the article/consumable may comprise an aerosol-cooling element which is adapted to cool the aerosol generated from the aerosol-forming substrate (by heat exchange) before being inhaled by the user.

The article/consumable may comprise a spacer element that defines a space or cavity between the aerosol-forming substrate and the downstream end of the consumable. The spacer element may comprise a cardboard tube. The spacer element may be circumscribed by the (paper) wrapping layer.

According to a third aspect of the present invention, there is provided a method of using the system according to the second aspect, the method comprising inserting the aerosol-forming article into the device; and heating the article using the heater of the device.

In some embodiments the method may comprise inserting the article into a cavity within a body of the device and penetrating the article with the heating element of the device upon insertion of the article.

According to a fourth aspect of the present invention, there is provided a method of operating a heat-not-burn device having a heater, said heater configured to operate at a first temperature in a first heating mode and a second temperature in a second heating mode, the second temperature being higher than the first temperature. The method further comprises sensing an ambient temperature and disabling the second heating mode when the measured ambient temperature exceeds a predetermined threshold.

Optionally, the method comprises receiving, by the controller, the ambient temperature from a sensor and disabling the second heating mode upon determining that the ambient temperature exceeds the predetermined threshold.

Advantageously, the method comprises disabling, by the controller, the second heating mode by switching the operation of the heater from the second heating mode to the first heating mode.

Conveniently, the first heating mode described in the method is a normal mode whereas, the second heating mode described in the method is a boost mode.

Advantageously, the method comprises generating, via output means, one of haptic, audio and visual feedback indicating a user that the controller has disabled the second heating mode.

Conveniently, the method comprises receiving user input, via a user input means, for switching to the second heating mode from the first heating mode, and generating a feedback, via the output means, indicating the user that the second heating mode is not enabled if the controller detected that the ambient temperature exceeds the predetermined threshold.

The invention includes the combination of the aspects and preferred features described except where such a combination is clearly impermissible or expressly avoided.

The skilled person will appreciate that except where mutually exclusive, a feature or parameter described in relation to any one of the above aspects may be applied to any other aspect. Furthermore, except where mutually exclusive, any feature or parameter described herein may be applied to any aspect and/or combined with any other feature or parameter described herein.

### SUMMARY OF THE FIGURES

So that the invention may be understood, and so that further aspects and features thereof may be appreciated, embodiments illustrating the principles of the invention will now be discussed in further detail with reference to the accompanying figures, in which:
Figure 1A is a schematic of a smoking substitute system;
Figure 1B is a schematic of a variation of the smoking substitute system of Figure 1A;
Figure 2A is a front view of a first embodiment of a smoking substitute system with the consumable engaged with the device;
Figure 2B is a front view of the first embodiment of the smoking substitute system with the consumable disengaged from the device;
Figure 2C is a section view of the consumable of the first embodiment of the smoking substitute system;
Figure 2D is a detailed view of an end of the device of the first embodiment of the smoking substitute system;
Figure 2E is a section view of the first embodiment of the smoking substitute system; and
Figure 3 is a flowchart illustrating a method for operating the system.

### DETAILED DESCRIPTION OF THE INVENTION

Aspects and embodiments of the present invention will now be discussed with reference to the accompanying figures. Further aspects and embodiments will be apparent to those skilled in the art. All documents mentioned in this text are incorporated herein by reference.

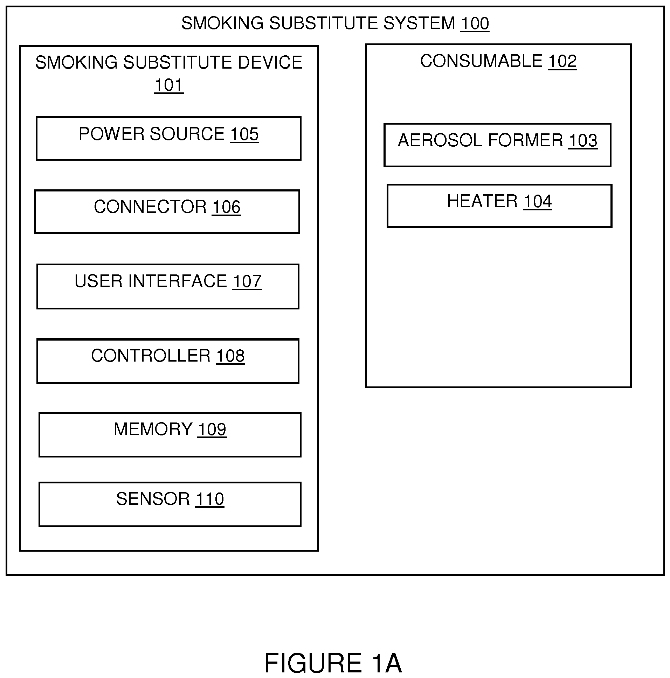

Figure 1A is a schematic providing a general overview of a smoking substitute system 100. The system 100 includes a substitute smoking device 101 and an aerosol-forming article in the form of a consumable 102, which comprises an aerosol former 103. The system is configured to vaporise the aerosol former by heating the aerosol former 103 (so as to form a vapour/aerosol for inhalation by a user).

In the illustrated system, the heater 104 forms part of the consumable 102 and is configured to heat the aerosol former 103. In this variation, the heater 104 is electrically connectable to the power source 105, for example, when the consumable 102 is engaged with the device 101. Heat from the heater 104 vaporises the aerosol former 103 to produce a vapour. The vapour subsequently condenses to form an aerosol, which is ultimately inhaled by the user. In some embodiment, the heater 104 is configured to operate in two operating modes. In particular, the heater 104 is configured to operate at a first temperature in a first heating mode and at a second temperature at a second heating mode, wherein the second temperature is higher than the first temperature. In the illustrated embodiment, the heater is configured to operate at a first temperature of 315°C, and at a second temperature at 345 °C.

The system 100 further comprises a power source 105 that forms part of the device 101. In other embodiments the power source 105 may be external to (but connectable to) the device 101. The power source 105 is electrically connectable to the heater 104 such that the power source 105 is able to supply power to the heater 104 (i.e. for the purpose of heating the aerosol former 103). Thus, control of the electrical connection of the power source 105 to the heater 104 provides control of the state of the heater 104. The power source 105 may be a power store, for example a battery or rechargeable battery (e.g. a lithium ion battery).

The system 100 further comprises an I/O module comprising a connector 106 (e.g. in the form of a USB port, Micro USB port, USB-C port, etc.). The connector 106 is configured for connection to an external source of electrical power, e.g. a mains electrical supply outlet. The connector 106 may be used in substitution for the power source 105. That is the connector 106 may be electrically connectable to the heater 104 so as to supply electricity to the heater 104. In such embodiments, the device may not include a power source, and the power source of the system may instead comprise the connector 106 and an external source of electrical power (to which the connector 106 provides electrical connection).

In some embodiments, the connector 106 may be used to charge and recharge the power source 105 where the power source 105 includes a rechargeable battery.

The system 100 also comprises a user interface (UI) 107. Although not shown, the UI 107 may include input means to receive commands from a user. The input means of the UI 107 allows the user to control at least one aspect of the operation of the system 100. The input means may, for example, be in the form of a button, touchscreen, switch, microphone, etc.

The UI 107 also comprises output means to convey information to the user. The output means may, for example, comprise lights (e.g. LEDs), a display screen, speaker, vibration generator, etc.

The system 100 further comprises a controller 108 and a memory 109 operatively coupled to the controller 108.that is configured to control at least one function of the device 101. In the illustrated embodiment, the controller 108 is a component of the device 101, but in other embodiments may be separate from (but connectable to) the device 101. The controller 108 is configured to sense the ambient temperature and in response disable the second heating mode if the ambient temperature exceeds a predetermined threshold temperature. In one aspect, the predetermined threshold temperature is previously determined and stored in the memory 109 of the device 101. Thus, the controller 108 is configured to control the operation of the heater 104 and, for example, may be configured to control the voltage applied from the power source 105 to the heater 104. The controller 108 may be configured to toggle the supply of power to the heater 104 between an on state, in which the full output voltage of the power source 105 is applied to the heater 104, and an off state, in which the no voltage is applied to the heater 104. In the illustrated embodiment, the predetermined threshold, or threshold temperature is 40°C.

Although not shown, the system 100 may also comprise a voltage regulator to regulate the output voltage from the power source 105 to form a regulated voltage. The regulated voltage may then be applied to the heater 104.
In addition to being connected to the heater 104, the controller 108 is operatively connected to the UI 107. Thus, the controller 108 may receive an input signal from the input means of the UI 107. Similarly, the controller 108 may transmit output signals to the UI 107. In response, the output means of the UI 107 may convey information, based on the output signals, to a user. The controller also comprises a memory 109, which is a non-volatile memory. The memory 109 includes instructions, which, when implemented, cause the controller to perform certain tasks or steps of a method.

Further, the system may also comprise a sensor 110 coupled with the controller 108 within the heat-not-burn device 101. The sensor 110 may be a temperature sensor mounted inside the device and configured to measure the ambient temperature of air external to the device 101. In addition, the sensor 110 may be configured to continuously monitor the ambient temperature and send the measured ambient temperature to the controller 108 so that the controller 108 is able to determine whether the second heating mode needs to be disabled or not.

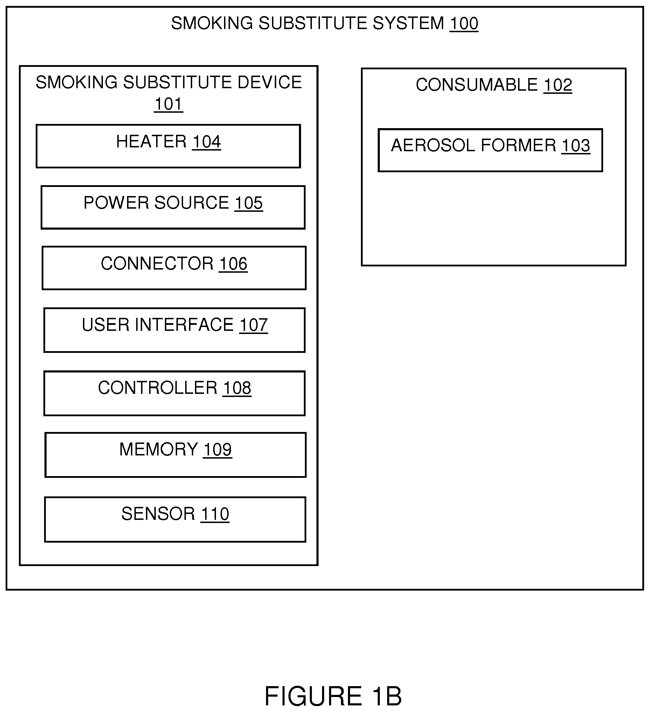

Figure 1B is a schematic showing a variation of the system 100 of Figure 1A. In the system 100' of Figure 1B, the heater 104 forms part of the device 101, rather than the consumable 102. In this variation, the heater 104 is electrically connected to the power source 105.

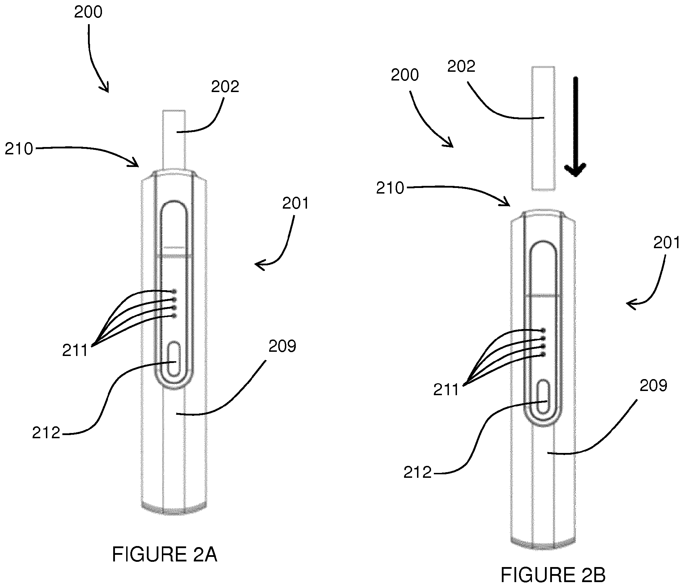

Figures 2A and 2B illustrate a heated-tobacco (HT) smoking substitute system 200. The system 200 is an example of the systems 100, 100' described in relation to Figures 1A or 1B. System 200 includes an HT device 201 that is configured to disable the second heating mode when the measured ambient temperature exceeds a predetermined threshold temperature and an HT consumable 202. The description of Figures 1A and 1B above is applicable to the system 200 of Figures 2A and 2B, and will thus not be repeated.

The device 201 and the consumable 202 are configured such that the consumable 202 can be engaged with the device 201. Figure 2A shows the device 201 and the consumable 202 in an engaged state, whilst Figure 2B shows the device 201 and the consumable 202 in a disengaged state.

The device 201 comprises a body 209 and cap 210. In use the cap 210 is engaged at an end of the body 209. Although not apparent from the figures, the cap 210 is moveable relative to the body 209. In particular, the cap 210 is slideable and can slide along a longitudinal axis of the body 209.

The device 201 comprises an output means (forming part of the UI of the device 201) in the form of a plurality of light-emitting diodes (LEDs) 211 arranged linearly along the longitudinal axis of the device 201 and on an outer surface of the body 209 of the device 201. A button 212 is also arranged on an outer surface of the body 209 of the device 201 and is axially spaced (i.e. along the longitudinal axis) from the plurality of LEDs 211.

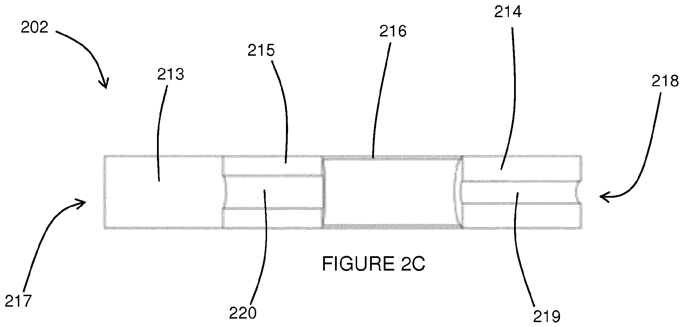

Figure 2C show a detailed section view of the consumable of 202 of the system 200. The consumable 202 generally resembles a cigarette. In that respect, the consumable 202 has a generally cylindrical form with a diameter of 7 mm and an axial length of 70 mm. The consumable 202 comprises an aerosol forming substrate 213, a terminal filter element 215, an upstream filter element 215 and a spacer element 216. In other embodiments, the consumable may further comprise a cooling element. A cooling element may exchange heat with vapour that is formed by the aerosol-forming substrate 213 in order to cool the vapour so as to facilitate condensation of the vapour.

The aerosol-forming substrate 213 is substantially cylindrical and is located at an upstream end 217 of the consumable 202, and comprises the aerosol former of the system 200. In that respect, the aerosol forming substrate 213 is configured to be heated by the device 201 to release a vapour. The released vapour is subsequently entrained in an airflow flowing through the aerosol-forming substrate 213. The airflow is produced by the action of the user drawing on a downstream 218 (i.e. terminal or mouth end) of the consumable 202.

In the present embodiment, the aerosol forming substrate 213 comprises tobacco material that may, for example, include any suitable parts of the tobacco plant (e.g. leaves, stems, roots, bark, seeds and flowers). The tobacco may comprise one or more of leaf tobacco, stem tobacco, tobacco powder, tobacco dust, tobacco derivatives, expanded tobacco, homogenised tobacco, shredded tobacco, extruded tobacco, cut rag tobacco and/or reconstituted tobacco (e.g. slurry recon or paper recon). For example, the aerosol-forming substrate 213 may comprise a gathered sheet of homogenised (e.g. paper/slurry recon) tobacco or gathered shreds/strips formed from such a sheet.

In order to generate an aerosol, the aerosol forming substrate 213 comprises at least one volatile compound that is intended to be vaporised/aerosolised and that may provide the user with a recreational and/or medicinal effect when inhaled. The aerosol-forming substrate 213 may further comprise one or more additives. For example, such additives may be in the form of humectants (e.g. propylene glycol and/or vegetable glycerine), flavourants, fillers, aqueous/non-aqueous solvents and/or binders.

The terminal filter element 214 is also substantially cylindrical, and is located downstream of the aerosol forming substrate 213 at the downstream end 218 of the consumable 202. The terminal filter element 214 is in the form of a hollow bore filter element having a bore 219 (e.g. for airflow) formed therethrough. The diameter of the bore 219 is 2 mm. The terminal filter element 214 is formed of a porous (e.g. monoacetate) filter material. As set forth above, the downstream end 218 of the consumable 202 (i.e. where the terminal filter 214 is located) forms a mouthpiece portion of the consumable 202 upon which the user draws. Airflow is drawn from the upstream end 217, thorough the components of the consumable 202, and out of the downstream end 218. The airflow is driven by the user drawing on the downstream end 218 (i.e. the mouthpiece portion) of the consumable 202.

The upstream filter element 215 is located axially adjacent to the aerosol-forming substrate 213, between the aerosol-forming substrate 213 and the terminal filter element 214. Like the terminal filter 214, the upstream filter element 215 is in the form of a hollow bore filter element, such that it has a bore 220 extending axially therethrough. In this way, the upstream filter 215 may act as an airflow restrictor. The upstream filter element 215 is formed of a porous (e.g. monoacetate) filter material. The bore 220 of the upstream filter element 214 has a larger diameter (3 mm) than the terminal filter element 214.

The spacer 216 is in the form of a cardboard tube, which defines a cavity or chamber between the upstream filter element 215 and the terminal filter element 214. The spacer 216 acts to allow both cooling and mixing of the vapour/aerosol from the aerosol-forming substrate 213. The spacer has an external diameter of 7 mm and an axial length of 14mm.

Although not apparent from the figure, the aerosol-forming substrate 213, upstream filter 215 and spacer 216 are circumscribed by a paper wrapping layer. The terminal filter 214 is circumscribed by a tipping layer that also circumscribes a portion of the paper wrapping layer (so as to connect the terminal filter 214 to the remaining components of the consumable 202). The upstream filter 215 and terminal filter 214 are circumscribed by further wrapping layers in the form of plug wraps.

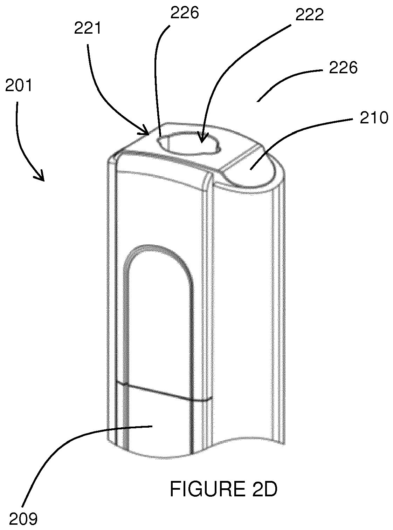

Returning now to the device 201, Figure 2D illustrates a detailed view of the end of the device 201 that is configured to engage with the consumable 202. The cap 210 of the device 201 includes an opening 221 to an internal cavity 222 (more apparent from Figure 2D) defined by the cap 210. The opening 221 and the cavity 222 are formed so as to receive at least a portion of the consumable 202. During engagement of the consumable 202 with the device 201, a portion of the consumable 202 is received through the opening 221 and into the cavity 222. After engagement (see Figure 2B), the downstream end 218 of the consumable 202 protrudes from the opening 221 and thus also protrudes from the device 201. The opening 221 includes laterally disposed notches 226. When a consumable 202 is received in the opening 221, these notches 226 remain open and could, for example, be used for retaining a cover in order to cover the end of the device 201.

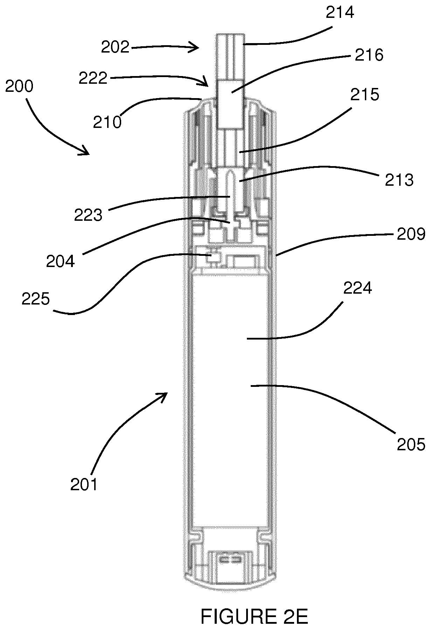

Figure 2E shows a cross section through a central longitudinal plane through the device 201. The device 201 is shown with the consumable 202 engaged therewith.

The device 201 comprises a heater 204 comprising heating element 223. The heater 204 forms part of the body 209 of the device 201 and is rigidly mounted to the body 209. In the illustrated embodiment, the heater 204 is a rod heater with a heating element 223 having a circular transverse profile. In other embodiments the heater may be in the form of a blade heater (e.g. heating element with a rectangular transverse profile) or a tube heater (e.g. heating element with a tubular form).

The heating element 223 of the heater 204 projects from an internal base of the cavity 222 along a longitudinal axis towards the opening 221. As is apparent from the figure, the length (i.e. along the longitudinal axis) of the heating element is less than a depth of the cavity 222. In this way, the heating element 223 does not protrude from or extend beyond the opening 221.

When the consumable 202 is received in the cavity 222 (as is shown in Figure 2E), the heating element 223 penetrates the aerosol-forming substrate 213 of the consumable 202. In particular, the heating element 223 extends for nearly the entire axial length of the aerosol-forming substrate 213 when inserted therein. Thus, when the heater 204 is activated, heat is transferred radially from an outer circumferential surface the heating element 223 to the aerosol-forming substrate 213.

The device 201 further comprises an electronics cavity 224. A power source, in the form of a rechargeable battery 205 (a lithium ion battery), is located in electronics cavity 224.

The device 201 includes a connector (i.e. forming part of an IO module of the device 201) in the form of a USB port 206. The connector may alternatively be, for example, a micro-USB port or a USB-C port for examples. The USB port 206 may be used to recharge the rechargeable battery 205.

The device 201 includes a controller (not shown) located in the electronics cavity 224. The controller comprises a microcontroller mounted on a printed circuit board (PCB). The USB port 206 is also connected to the controller 208 (i.e. connected to the PCB and microcontroller).

The controller 208 is configured to control at least one function of the device 201. For example, the controller 208 is configured to control the operation of the heater 204. Such control of the operation of the heater 204 may be accomplished by the controller toggling the electrical connection of the rechargeable battery 205 to the heater 204. For example, the controller 208 is configured to control the heater 204 in response to a user depressing the button 212. Depressing the button 212 may cause the controller to allow a voltage (from the rechargeable battery 205) to be applied to the heater 204 (so as to cause the heating element 223 to be heated).

In one aspect, the controller 208 is configured to control the operating modes of the heater 204. Operating modes of the heater 204, may be for example, a first heating mode and a second heating mode. The controller 208 is configured to disable the second heating mode by comparing measured ambient temperature with the predetermined threshold temperature. Precisely, the controller 208 is configured to receive the measured ambient temperature of the device 201, from the sensor 110 and thereby disables the second heating mode when the measured ambient temperature is determined to have exceeded the predetermined threshold temperature. The heater 204 of the device 201 operates at a first temperature in a first heating mode and at a second temperature in a second heating mode. In one example, the second temperature may be higher than the first temperature i.e. the first mode may be defined as a normal operating mode where the consumable 202 is heated at a steady temperature and the second mode may be defined as a boost heating mode where the consumable 202 is heated up more quickly when the heater is operating at a higher temperature. In the illustrated embodiment, the heater is configured to operate at a first temperature of 315°C, and at a second temperature at 345°C. The predetermined threshold, or predetermined threshold temperature, is 40°C.

The controller 208 is configured to constantly receive input, in the form of measured ambient temperature, from the sensor 110 and disable the second heating mode when the ambient temperature is determined to have exceeded a predetermined threshold. Such determination is performed using the memory 109 which stores a predetermined threshold temperature, and when the measured ambient temperature exceeds said predetermined threshold temperature the second heating mode is to be disabled. In particular, the predetermined threshold temperature stored in the memory 109 defines a cut off, and when the measured temperature exceed said predetermined threshold temperature the consumable shall not be heated.

In one aspect, the device 201 receives a user input, via the user interface, to switch the device 201, more specifically the heater 204, from operating in the first heating mode to the second heating mode, the controller 208 compares the detected ambient temperature with the predetermined threshold temperature that is stored in the memory 109 and accordingly the controller 208 may or may not switch from the first heating mode to the second heating mode. For example, if the measured ambient temperature is determined to have exceeded the predetermined threshold temperature, the controller 208 does not permit the heater form switching to operate in the second heating mode and therefore heating resumes in the first heating mode. On the other hand, if the ambient temperature is determined to be below the predetermined threshold temperature, then the controller 208 permits the device 201, e.g. the heater 204,to operate in the second heating mode.

In the illustrative aspect, when the heater is already operating in the second heating mode, upon determining the measured ambient temperature has exceeded the predetermined threshold, the controller 208 is configured to disable the second heating mode by switching the operation of the heater 204 from the second heating mode to the first heating mode, i.e. by operating the device at the first temperature instead of second temperature. In other embodiments, instead of switching the operation heater to a lower temperature, e.g. the first temperature, the controller may cease heating altogether. This allows the heater and consumable to cool down faster.

The controller 208 is also configured to control the LEDs 211 in response to (e.g. a detected) a condition of the device 201 or the consumable 202. For example, the controller may control the LEDs to indicate whether the device 201 is in an on state or an off state (e.g. one or more of the LEDs may be illuminated by the controller when the device is in an on state). In addition, the controller 208 may control the LEDs to indicate that the second mode is disabled when the measured ambient temperature is determined to have exceeded the pre-determined threshold temperature. The device 201 additionally includes other output means such as haptic sensor, audio sensors etc. to provide haptic/audio feedback to the user indicating the second mode has been disabled.

The device 202 comprises a further input means (i.e. in addition to the button 212) in the form of a puff sensor 225. The puff sensor 225 is configured to detect a user drawing (i.e. inhaling) at the downstream end 218 of the consumable 202. The puff sensor 225 may, for example, be in the form of a pressure sensor, flowmeter or a microphone. The puff sensor 225 is operatively connected to the controller 208 in the electronics cavity 224, such that a signal from the puff sensor 225, indicative of a puff state (i.e. drawing or not drawing), forms an input to the controller 208 (and can thus be responded to by the controller 208).

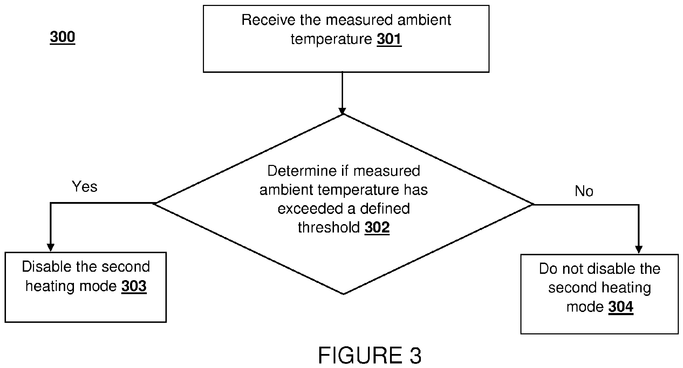

Figure 3 illustrates flowchart of method for disabling second heating mode when the detected ambient temperature is determined to have exceeded the predetermined threshold temperature.

As illustrated in figure 3, the method 300 includes one or more blocks implemented by the controller 208 of the device 201. The method 300 may be described in general context of controller executable instructions. Generally, controller executable instructions may include routines, programs, objects, components, data structures, procedures, modules, and functions, which perform particular functions or implement particular abstract data types.

The order in which the method 300 is described is not intended to be construed as a limitation, and any number of the described method blocks can be combined in any order to implement the method 300. Additionally, individual blocks may be deleted from the method 300 without departing from the scope of the subject matter described herein. Furthermore, the method 300 can be implemented in any suitable hardware, software, firmware, or combination thereof.

At block 301, the controller 208 receives a measured ambient temperature from the temperature sensor 110. It is to be noted that though the step of receiving a user input for changing the device from first heating mode to the second heating mode is not explicitly shown but it may be preceded before the step 301. At block 302, the controller compares the measured ambient with the predetermined threshold temperature stored in memory 109 and base thereon determine if the measured ambient temperature has exceeded the threshold temperature. In particular, when the controller 208 receive a user request for switching the device 201 from the first heating mode to the second heating mode, the controller 208 compares the measured ambient temperature, received at that particular moment from the sensor 110, with the predetermined threshold temperature and accordingly takes the decision as discussed in blocks 303 and 304.

At block 303, the controller 208 moves along "YES" path to disable the second heating mode or in other words to prevent enabling the second heating mode, i.e. when the determined ambient temperature is found exceeding the predetermined threshold stored in the memory 109. In addition, the controller 208 indicates the user that the second mode is not available, e.g. disabled, through various output means. Precisely, the controller 208 may be coupled with output means configured for generating one of haptic feedback, audio and visual feedback to indicate a user that the controller 208 has disabled the second heating mode.

At block 304, the controller 208 moves along "NO" path and do not disable the second heating mode, e.g. to enable or continue to enable the second heating mode, as the measured ambient temperature is not found exceeding the predetermined threshold.

The flowchart of method do not explicitly disclose, however in one exemplary embodiment, the controller 208 is configured for disabling the second heating mode by switching the operation of the heater from the second heating mode to the first heating mode. Further, the flowchart also does not explicitly disclose that the first heating mode and the second heating mode, however it is to be acknowledged that the the first mode may be a normal mode where the consumable 202 is heated at a steady temperature and the second mode may be a boost mode where the consumable 202 is heated quickly at a higher temperature.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the words "have", "comprise", and "include", and variations such as "having", "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means, for example, +/- 10%.

The words "preferred" and "preferably" are used herein refer to embodiments of the invention that may provide certain benefits under some circumstances. It is to be appreciated, however, that other embodiments may also be preferred under the same or different circumstances. The recitation of one or more preferred embodiments therefore does not mean or imply that other embodiments are not useful, and is not intended to exclude other embodiments from the scope of the disclosure, or from the scope of the claims.

## Claims

1. A heat-not-burn (HNB) device comprising:
a heater configured to operate at a first temperature in a first heating mode and a second temperature in a second heating mode, the second temperature is higher than the first temperature; and
a sensor configured to measure an ambient temperature;
wherein the heat-not-burn device is configured to disable the second heating mode when the measured ambient temperature exceeds a predetermined threshold.

2. The device according to claim 1, further comprises a controller electrically connected with the heater and the sensor, the controller is configured to receive the measured ambient temperature from the sensor and to disable the second heating mode upon determining that said measured ambient temperature exceeds the predetermined threshold.

3. The device according to claim 2, wherein the controller is configured to disable the second heating mode by switching the operation of the heater from the second heating mode to the first heating mode.

4. The device according to any one of the preceding claims, further comprises an output means configured to output one or more of a haptic feedback, an audio feedback and a visual feedback upon disabling of the second heating mode.

5. The device according to any preceding claim, wherein the device further comprises a user interface, wherein upon receiving a user input at the user interface the heater is configured to switch between operating in the second heating mode and the first heating mode.

6. The device according to claim 5, wherein the heater is prevented from switching from operating in the first operating mode to the second operating mode if the measured ambient temperature exceeds the predetermined threshold.

7. The device according to any one of the preceding claims, wherein the first temperature ranges from 300°C to 330°C and the second temperature ranges from 330°C to 360°C, or the first temperature ranges from 305°C to 325°C and the second temperature ranges from 335°C to 355°C.

8. The device according to any one of the preceding claims, wherein the predetermined threshold is at least 40°C or at least 35°C.

9. A smoking substitute system, comprising the heat-not-burn device according to any one of claims 1 to 8 and an aerosol-forming article for use with the heat-not-burn device.

10. A method of operating a heat-not-burn device having a heater, said heater is configured to operate at a first temperature in a first heating mode and a second temperature in a second heating mode, the second temperature is higher than the first temperature, the method comprises:
measuring an ambient temperature; and
disabling the second heating mode when the measured ambient temperature exceeds a predetermined threshold.

11. The method according to claim 10, wherein disabling the second heating mode comprises switching the operation of the heater from the second heating mode to the first heating mode.

12. The method according to claim 10 or claim 11, further comprises outputting one or more of a haptic feedback, an audio feedback and a visual feedback upon disabling the second heating mode.

13. The method according to any one of the claims 10 to 12, further comprises receiving user input; and switching the operation of the heater between the first heating mode and the second heating mode upon receiving said user input.

14. The method according to claim 13, further comprises preventing the heater from switching form operating in the first operating mode to the second operating mode if the measured ambient temperature exceeds the predetermined threshold.

15. The method according to claim 14, outputting one or more of a haptic feedback, an audio feedback and a visual feedback when the heater is prevented from switching from operating in the first operating mode to the second operating mode.
